# EUROPEAN PATENT SPECIFICATION

(11) **EP 0 626 383 B1**
(45) Date of publication and mention of the grant of the patent: **16.09.1998**
(21) Application number: 94302770.6
(22) Date of filing: 19.04.1994
(51) Int. Cl.: C07D 487/22, A61K 31/495

(54) **Palau'amine: a pharmacologically active compound from a sponge**
Palau'amin: eine pharmakologisch aktive Verbindung aus einem Schwamm
Palau'amine: composé à activité pharmacologique d'une éponge

(30) Priority: 20.04.1993 GB 9308111
(43) Date of publication of application: 30.11.1994
(73) Proprietor: PHARMA MAR, S.A., 28760 Tres Cantos, Madrid (ES)
(72) Inventor: Kinnel, Robin Bryan, Clinton New York 13323 (US); Gehrken, Henning-Peter, D-6701 Maxdorf (DE); Scheuer, Paul Joseph, Honolulu Hawaii 96822 (US); Gravalos, Dolores Garcia, E-28006 Madrid (ES); Faircloth, Glynn Thomas, Cambridge Massachussets 02140 (US)
(74) Representative: Ruffles, Graham Keith

(56) References cited:
- J. AM. CHEM. SOC. vol. 115, no. 8 , 1993 pages 3376 - 3377 R.B. KINNEL ET AL. 'Palau'amine: A Cytotoxic and Immunosuppressive Hexacyclic Bisguanidien Antibiotic from the Sponge Stylotella agminata'
- CHEMICAL ABSTRACTS, vol. 112, no. 21, 21 May 1990, Columbus, Ohio, US; abstract no. 191246y, M. ENDO 'Pharmacologically active substances from Palauan marine sponges' page 4 ;
- CHEMICAL ABSTRACTS, vol. 110, no. 23, 5 June 1989, Columbus, Ohio, US; abstract no. 204918e, M. ENDO 'Pharmacologically active substances from Palauan sponges' page 3 ;

## Description

This invention relates to an active compound, having cytotoxic and other properties, which has been isolated from a Pacific sponge.

Guanidine is a familiar structural feature in marine natural products. Examples range from simple arginine derivatives to complex polycycles as, e.g., saxitoxin and tetrodotoxin. We have now isolated and identified a hexacyclic bisguanidine, hereinafter referred to as palau'amine, from a sponge Stylotella agminata, collected in the Western Caroline Islands. Aqueous extracts of the sponge, first collected in 1977, and recollected in November 1991 at a depth of -5 to -50 m near Wonder Channel and Rock Islands, Republic of Belau, had substantial activity against Gram-negative and Gram-positive organisms and showed remarkable resistance to fungal growth on prolonged storage.

According to the invention, therefore, there is provided palau'amine of the formula: 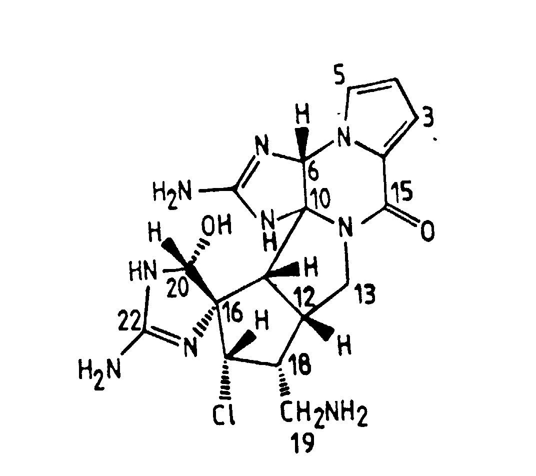 in the form an addition salt thereof.

The antitumor activities of this compound have been determined "in vitro" in cell cultures of human lung carcinoma A-549 and human colon carcinoma HT-29. The procedure was carried out using the methodology described by Raymond J. Bergeron et al. Biochem.Bioph. Res. Comm. 1984, 121(3), 848-854 and by Alan C. Schroeder et al. J. Med. Chem. 1981, 24 1078-1083. The compound also shows antibiotic and antifungal activity.

Therefore, the present invention also provides a method of treating any mammal affected by a malignant tumor sensitive to compounds above described, which comprises administering to the affected individual a therapeutically effective amount of these compounds or a pharmaceutically composition thereof; and a method of treating fungal infections in mammals, comprising administering to a patient in need of such treatment, an antifungal effective amount of the compounds described in the present invention; and a method of treating mammals in order to avoid immune response with an effective amount of the compounds described in the present invention.

The present invention also relates to pharmaceutical preparations which contain as active ingredient a salt of palan'amine as well as the process for its preparation.

Examples of pharmaceutical compositions include any solid (tablets, pills, capsules, granules, etc.) or liquid (solutions, suspensions or emulsions) suitable composition for oral, topical or parenteral administration, and they may contain the pure compound or in combination with any carrier or other phramcologically active compounds. These compositions may need to be sterile when administered parenterally.

The correct dosage of a pharmaceutical composition of these compounds will vary according to the particular formulation, the mode of application and particular situs, host and tumor being treated. Other factors like age, body weight, sex, diet, time of administration, rate of excretion, condition of the host, drug combinations, reaction sensitivities and severity of disease shall be taken in account. Administration can be carried out continuously or periodically within the maximum tolerated dose.

Palau'amine was isolated from the sponge as follows. Extraction of the lyophilized sponge (600 g) with MeOH (6 L) and dissolution of the water soluble residue after evaporation yielded 900 ml of an aqueous extract. [The dust from the lyophilized sponge caused a powerful allergic reaction, which entailed severe shortness of breath for about four hours; the effect largely disappeared within 24 hours]. Ion exchange chromatography of a portion (2/9) on Cellex CM with stepwise increasing concentrations of NaCl resulted in elution of the antibiotic activity (monitored by microbial assay against S. aureus) in the 0.5 M and to a lesser extent, in the 1.0 M fractions. Repeated LH-20 chromatography (MeOH) of the 0.5 fraction, after desalting by dissolving in EtOH, furnished essentially pure palau'amine, presumably as the hydrochloride, (14 mg, 0.01% dry weight) as an optically active, off-white amorphous powder that decomposed prior to melting. Further purification of palau'amine could be effected by HPLC [YMC aqueous C18, H₂O/MeCN (90:10), 0.1% TFA]. Although palau'amine is quite stable in acid, it decomposes rapidly >pH6.5, so that the free base could not be prepared.

Monoprotonated palau'amine has composition C₁₇H₂₂ClN₉O₂, which is based on high resolution mass spectral data (HRFABMS 420.1669 [MH+]. Δ0.6 mmu), on the isotopic cluster characteristic of one chlorine substituent, and on the ¹³C NMR spectrum. The IR spectrum showed O-H and N-H bands (3350 cm⁻¹, broad), an amide (1658 cm⁻¹) and an absorption at 1700 cm⁻¹ characteristic of a guanidine hydrochloride. (Goto, T.; Nakanishi, K; Ohashi, M. Bull. Chem. Soc. Japan 1957, 30 723-725). UV (MeOH) λₘₐₓ 272 (ε 7900), 224 nm (7800) and ¹H NMR data resembled those reported for phakellin (2, R₁=R₂=H) (Sharma, G.; Magdoff-Fairchild, B.J. Org. Chem. 1977, 42 4118-4142; Sharma, G.M.; Burkholder, P.R. J.Chem. Soc. Chem. Commun. 1971, 151-152). Full NMR data (Table 1) revealed its characteristic guanidino pyrrolopyrazinone. Distinctly new features included a C₆H₈ portion (A), confirmed by COSY and decoupling experiments, a guanidine carbon (159.9 ppm, C22), a methine (83.5 ppm, C20) and a quaternary carbon (72.1 ppm, C16).

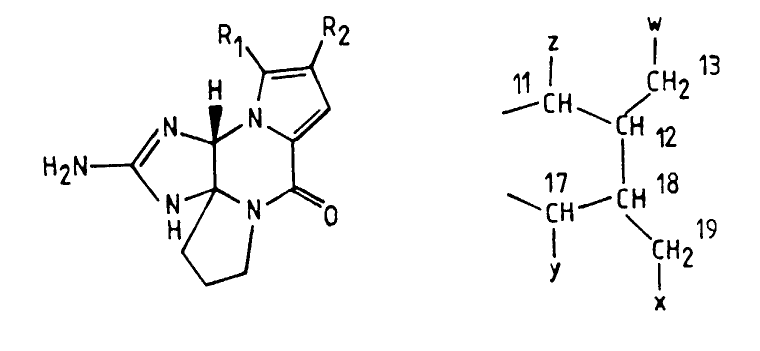

Fragment A replaces the trimethylene unit in phakellin. The C13 methylene is attached to the amide nitrogen (ω in A). The chemical shifts of the methylene carbon and protons, which show HMBC contours to C10 and C15, are analogous to those in the phakellins. Terminus z is C10; correlations are observed from H11 to C10 and C6.

The correct regiochemistry of the remaining hetero functions was ascertained next. The carbon (74.0 ppm) and proton (4.35 ppm) shifts for C17 indicate that y is oxygen or chlorine. The shifts were unaffected by acetylation; nor was there any substantial change in that proton resonance when the ¹H NMR spectrum was determined in trifluoroacetic acid. Thus, y must be chlorine, and, since H17 is a doublet, C16 must be a quaternary carbon.

Acetylation of 1 with Ac₂O in pyridine resulted in a mixture from which no simple mono derivatives was isolated. However, aqueous Ac₂O/NaOAc yielded a monoacetyl derivative. NMR experiments (COSY, HMQC, HMBC in D₂O and DMSO-d⁶) demonstrated that an acetamide had formed from a primary amine (x in A) attached to C19 methylene.

**Table I.**

| ¹H and ¹³C NMR Data for Palau'amine in D₂O | | | | |
|---|---|---|---|---|
| Carbon | ¹³C,ppm^{a} | Multiplicity | ¹H,ppm^{b} | Multiplicity |
| 2 | 122.5 | s | | |
| 3 | 115.6 | d | 6.85 | dd,J=3.9,1.5 |
| 4 | 113.8 | d | 6.35 | dd,J=3.9,2.8 |
| 5 | 125.2 | d | 6.99 | dd,J=2.8,1.5 |
| 6 | 69.0 | d | 6.33 | s |
| 8 | 159.5 | s | | |
| 10 | 80.8 | s | | |
| 11 | 56.3 | d | 3.08 | d,J=14.1 |
| 12 | 41.8 | d | 2.52 | dddd |
| 13 | 46.1 | t 3.28 | 3.96 | dd,J=7.3,10.4 dd,J=10.3,10.4 |
| 15 | 157.8 | s | | |
| 16 | 72.1 | s | | |
| 17 | 74.0 | d | 4.35 | d,J=7.9 |
| 18 | 48.6 | d | 2.47 | dddd |
| 19 | 41.9 | t | 3.32 | dd,J=13.2,7.0 |
| | | 3.24 | dd,J=13.2,7.0 | |
| 20 | 83.7 | d | 5.96 | s |
| 22 | 157.9 | s | | |

| | | | | |
|---|---|---|---|---|
| ^{a} at 125 MHz referred to external dioxane: | | | | |
| ^{b} at 50 MHz, HDO signal at 4.63 ppm. | | | | |

Presence of an hydroxyl was inferred by loss of water from the molecular ion in the MS-MS spectrum. MH⁺-18,26%. Also observed were peaks at MH⁺-59, -83, and -142; a peak for guanidinium (m/z 60, 51%) and for acylpyrrolium (m/z 94, 35%) were the other strong peaks.

The remaining structural features were elucidated with the aid of HMBC data. Both H11, a clean doublet, J=14.1 Hz, and H17 showed correlations to a quaternary carbon at 72 ppm (C16), thus C16 must be attached to both carbons. H11 also correlates to a methine carbon at 83.5 ppm (C20) and to five other carbons. The proton attached to C20 is a singlet in D₂O and a broadened doublet in DMSO-d₆. Thus C20 is vicinal to an amide or guanidine NH group. Furthermore, H20 shows an HMBC to C16, C11 and C22, therefore C20 must be a carbinolamine which is part of a ring containing the guanidine. Structure 1 of palau'amine is consistent with all of these data.

Relative stereochemistry can be deduced from nOe's and interproton coupling. H11 and H6 show positive nOe and ROESY correlations. The bicyclo[3-3.0]azaoctane ring is assuredly cis fused, while inspection of models and comparison of coupling constants suggests that the H12, H18 and H17 are all cis to one another. The NH proton that is coupled to H20 shows a clear ROESY correlation to H6. This is only possible if C20 is β-oriented. H20 and H17 show long-range COSY and ROESY correlations, which indicates syn geometry.

Palau'amine is reasonably non-toxic and has an LD₅₀ (i.p. in mice) of 13 mg/Kg.

The compound is active against the following tumour cells at the inhibiting concentrations noted:-

| Cell | IC₅₀ |
|---|---|
| P-388 | 0.1µg |
| A-549 | 0.2µg |
| HT-29 | 2 µg |
| KB | 10 µg |

Further, palau'amine showed antibiotic activity against S.aureus and B.subtilis at 10 µg/disk, and antifungal activity giving a 24 mm zone against Penicillium notatum at 50 µg/disk.

In the mixed lymphocyte reaction (MLR) palau'amine showed an IC₅₀ <18ng/ml, while the cytotoxicity assay against a primary culture of murine lymphocytes showed an IC₅₀ of 1.5 µg/ml.

We have also identified the following known compounds in extracts of this sponge: sceptrin, hymenidin, oroidin, dibromophakellin, hymenialdisine, hymenin, and "the yellow compound". There are also at least three less active, brominated derivatives of palau'amine present, as well as the analog to dibromoisophakellin.

## Claims

1. Palau'amine of the formula: 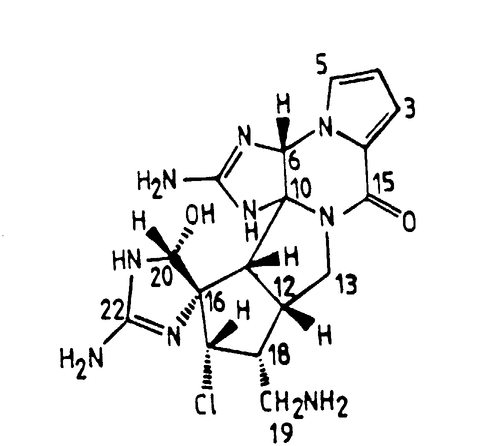 in the form of an acid addition salt thereof.

2. A pharmaceutical composition comprising palau'amine in association with a pharmaceutical carrier or diluent.

3. The use of palau'amine in the manufacture of an antitumoral pharmaceutical composition.

4. The use of palau'amine in the manufacture of an antifungal pharmaceutical composition.

5. The use of palau'amine in the manufacture of an immuno-suppressive pharmaceutical composition.

## Patentansprüche

1. Palau'amin der Formel: 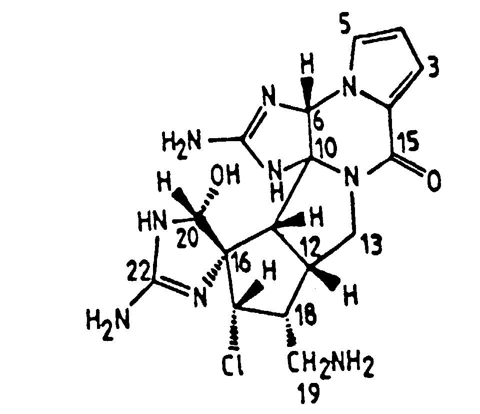 in der Form seines Säureadditionssalzes.

2. Pharmazeutische Zusammensetzung umfassend Palau'amin in Verbindung mit einem pharmazeutischen Träger oder Verdünnungsmittel.

3. Verwendung von Palau'amin bei der Herstellung einer pharmazeutischen Antitumorzusammensetzung.

4. Verwendung von Palau'amin bei der Herstellung einer pharmazeutischen Antipilzzusammensetzung.

5. Verwendung von Palau'amin bei der Herstellung einer pharmazeutischen immunsuppressiven Zusammensetzung.

## Revendications

1. Palau'amine, répondant à la formule: 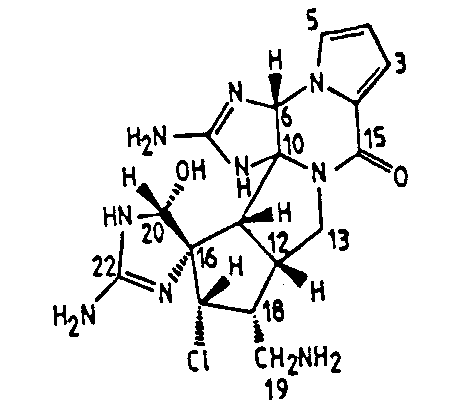 sous la forme d'un sel d'addition d'acide de celle-ci.

2. Composition pharmaceutique, contenant de la palau'amine associée à un support ou diluant pharmaceutique.

3. Utilisation de palau'amine dans la fabrication d'une composition pharmaceutique antitumorale.

4. Utilisation de palau'amine dans la fabrication d'une composition pharmaceutique antifongique.

5. Utilisation de palau'amine dans la fabrication d'une composition pharmaceutique immunosuppressive.
